# EUROPÄISCHE PATENTSCHRIFT

(11) **EP 2 904 979 B1**
(45) Veröffentlichungstag und Bekanntmachung des Hinweises auf die Patenterteilung: **23.08.2017**
(21) Anmeldenummer: 15153601.8
(22) Anmeldetag: 03.02.2015
(51) Int. Cl.: A61B 17/02, A61B 17/29

(54) **Montageverfahren für ein mikrochirurgisches Instrument**
Method for assembling a microsurgical instrument
Procédé de montage d'instrument micro-chirurgical

(30) Priorität: 10.02.2014 DE 102014101601
(43) Veröffentlichungstag der Anmeldung: 12.08.2015
(73) Patentinhaber: Karl Storz GmbH & Co. KG, 78532 Tuttlingen (DE)
(72) Erfinder: Merz, Robin, 78532 Tuttlingen (DE); Stefan, Jochen, 78532 Tuttlingen (DE)

(56) Entgegenhaltungen:
- DE-A1- 19 920 869
- DE-A1-102006 038 516
- DE-A1-102012 007 645
- US-A1- 2010 130 817

## Beschreibung

Die vorliegende Erfindung betrifft ein Montageverfahren für ein mikrochirurgisches Instrument, insbesondere einen abwinkelbaren Retraktor.

Mikrochirurgische Instrumente, beispielsweise Retraktoren, sind häufig zerlegbar ausgeführt, um einfacher gereinigt werden zu können: Dabei können der Schaft, die Handhabe und die Retraktions- oder eine andere Arbeitsstruktur voneinander trennbar sein. Es ist bekannt, die Verbindung von Schaft und der Retraktions- oder der anderen Arbeitsstruktur, etwa ein Werkzeugkopf, durch eine lösbare Kopplungsvorrichtung wie einen Bajonettverschluss herzustellen.

Aus der DE 10 2006 038 516 A1 ist ein Rohrschaftinstrument bekannt, das eine in einem Schaft geführte Betätigungsstange aufweist, deren proximales Ende mit einem Betätigungselement verbunden werden kann und deren distales Ende zum Betätigen des Werkzeugs mit dem Werkzeugkopf gekoppelt ist.

Am Werkzeugkopf liegt eine Hülse vor, die lösbar über eine Zapfen-Schlitz-Verbindung mit gekrümmter Führungsbahn mit dem hohlen Schaft gekoppelt wird.

Zapfen-Schlitz-Verbindungen oder Bajonettverbindungen bieten zwar den Vorteil, dass sie intuitiv und schnell zu bedienen sind, sie neigen aber unter Torsionsbelastung dazu, sich selbsttätig zu lösen, was insbesondere bei Werkzeugköpfen mit vergleichweise hohem Gewicht bzw. Werkzeugköpfen mit großer radialer Ausladung wie einer Retraktionsstruktur ein Sicherheitsrisiko beim Bedienen darstellt. Es wurden daher Ansätze entwickelt, die das unbeabsichtigte Lösen einer Bajonettverbindung erschweren sollen.

Eine solche Bajonettverbindung, die zum Verbinden zweier rohrförmiger Instrumententeile vorgesehen ist, wird in der DE 197 07 373 C1 offenbart. Dort ist an einer distalen Instrumentenhälfte eine Bajonetthülse vorgesehen, die einen L-förmigen Schlitz aufweist, wobei in die Bajonetthülse ein Bajonetteinsatz, der radial und längsaxial abstehende Eingriffsnasen hat, eingeführt wird. Distal "hinter" der Bajonetthülse ist ein Verriegelungsstück angeordnet, das durch eine Feder in proximale Richtung gedrückt wird und eine stirnseitige Ausnehmung hat, deren Form mit den Nasen des Bajonetteinsatzes korrespondiert. Befindet sich der Bajonetteinsatz in seiner Sperrdrehstellung, so greifen die Nasen in die Ausnehmung ein und das Verriegelungsstück federt zurück, wodurch weiteres Verdrehen des Bajonetteinsatzes unterbunden wird. Durch die dort offenbarte Lösung kann das Verdrehen nur erschwert werden, gänzlich verhindert werden kann es nicht, da, wenn das Drehmoment auf den Schaft hoch genug ist, das Verriegelungsstück entgegen der Federbelastung bewegt werden kann, wodurch der Eingriff mit der Ausnehmung aufgelöst wird.

Es wurden daher noch weiter modifizierte Bajonettververbindungen entwickelt, die das unbeabsichtigte Verdrehen sicherer unterbinden sollen.

So betrifft die DE 10 2012 007645 eine Gelenkeinrichtung für ein medizinisches Instrument, bei der eine Handhabe über einen Schaft mittels eines Bajonetts an ein Gelenk gekoppelt ist. Im Schaft sind eine Betätigungsstange und ein Innenschaft geführt, die zum Bedienen von Werkzeugfunktionen vorgesehen sind. Der (Außen-) Schaft ist über die Bajonettverbindung mit einem Gelenkkörper verbunden. Hierzu ist an dem proximalen Ende des Gelenkkörpers eine Hülse angeordnet, die an ihrem proximalen Ende radial abstehende Klauen hat. Die Hülse hat eine längsaxiale Schlitzung, durch die ein verschiebefest auf dem Innenschaft befestigter radial abstehender Nocken tritt. Dieser hat eine größere Länge und erstreckt sich radial weiter, als die Hülse dick ist. Der Nocken kann zusammen mit dem Innenschaft in eine distale Endlage gebracht werden, in der er in einer korrespondierenden Nische eines Flanschs der Hülse an dem Gelenkkörper aufgenommen ist. Der (Außen-) Schaft hat an seinem distalen Ende einen L-förmigen Schlitz, der zum Koppeln über die Hülse des Gelenkkörpers gestreift wird, wobei die Klauen in Eingriff mit dem Schlitz gebracht werden.

In der Sperrposition des Bajonetts fluchten die längsaxialen Schlitzabschnitte und die Nische des Flanschs, in der der Nocken aufgenommen ist, so dass der Nocken aus der Nische nach proximal in die längsaxialen Schlitzabschnitte bewegt werden kann, wodurch eine Verdrehsicherung erreicht wird. Die Verdrehsicherung dort kann jedoch erst wirksam werden, wenn der Nocken aus der Nische herausgefahren ist und mit den beiden längsaxialen Schlitzabschnitten in Eingriff steht.

Ausgehend von diesem Stand der Technik liegt der vorliegenden Erfindung die Aufgabe zu Grunde, ein mikrochirurgisches Instrument, das mit Bajonettverschluss arbeitet, so montieren zu können, dass in jedem Betriebszustand des Instruments eine wirksame Verdrehsicherung der Bajonettverbindung gegeben ist.

Diese Aufgabe wird durch das Montageverfahren für ein mikrochirurgisches Instrument mit den Merkmalen des unabhängigen Anspruchs 1 gelöst.

Weiterbildungen des Verfahrens werden jeweils durch die Unteransprüche beschrieben.

Anders als bekannte mikrochirurgische Instrumente, die über eine ähnliche werkzeugnahe Verdrehsicherung verfügen, wird durch die Kombination der werkzeugnahen Verdrehsicherung mit dem Eingriff der Klemmsteine der Handhabe auf der Abplattung der Betätigungsstange eine redundante zweite Verdrehsicherung geschaffen, die schon dann aktiv ist, wenn die werkzeugnahe Verdrehsicherung noch nicht aktiv ist. Somit kann effektiv verhindert werden, dass sich die Bajonettverbindung kurz nach dem Schließen aufdreht, ohne dass zur Aktivierung der "handhabenahen" Verdrehsicherung aktives Tun des Verwenders nötig wäre. Die "handhabenahe" Verdrehsicherung wird allein durch in Eingriff bringen des Klemmsteins der Handhabe mit der Abplattung der Betätigungsstange erreicht, was quasi "automatisch" beim Einstecken der Betätigungsstange in die Handhabe erfolgt. Damit der Nocken sperrend in beiden Längsnuten, d. h. die Längsnut der beiden Bajonettelemente, eingreifen kann, muss der Nocken beide Bajonettelemente zumindest teilweise der Dicke nach durchdringen. Es kann auch mehr als ein Nocken zum Einsatz kommen, wobei jeder Nocken in einer eigenen Längsnut geführt sein kann.

Der Nocken kann ferner verschiebefest mit der Betätigungsstange verbunden sein, wobei im Schritt f) die Betätigungsvorrichtung der Handhabe betätigt und dadurch die Betätigungsstange längsaxial bewegt und der Nocken zwangsgekoppelt mitbewegt wird.

Hierdurch kann der Nocken durch die gewohnten Bewegungsabläufe unter Verwendung der Handhabe verschoben werden, ohne dass dazu mit einer Hand der Nocken direkt bewegt werden müsste, was zu einer verbesserten Ergonomie beiträgt.

Ferner können die Längsschlitze der Bajonettelemente und einer oder mehrere Eingriffskörper der Bajonettverbindung in einem Freigabezustand der Bajonettverbindung angular versetzt vorliegen, wobei im Schritt b) ein distales Ende eines dem Schaft zugeordneten Bajonettelements an dem Nocken zur Anlage gebracht wird, und der Nocken beim Verschieben des Schafts mitverschoben wird.

Durch die in Bezug auf die Längsachse angular versetzte Anordnung des/der Eingriffskörper und der Längsschlitze kann erreicht werden, dass die beiden Längsschlitze der Bajonettelemente im Sperrzustand fluchtend ausgerichtet sind. Ferner kann der Nocken beim Überschieben des Schafts von einem Abschnitt des distalen Endes des dem Schaft zugeordneten Bajonettelements mitgeschoben werden, während der/die Eingriffskörper in den Längsschlitz/die Längsschlitze eingeführt werden.

Schließlich kann an einem proximalen Ende des Werkzeugkopfs ein Anbindungsabschnitt vorliegen, der eine Nische aufweist, die mit einer Größe und Position des Nockens korrespondiert. Dabei kann vor dem Schritt c) der Schritt b'), Verschieben des Nockens in eine distale Verschiebeendposition, dort Aufnahmen des Nockens in der Nische und dadurch Freigeben eines Verdrehfreiheitsgrades der Bajonettelemente, ausgeführt werden. Die Nische muss dabei mindestens so lang sein wie der Nocken, so dass die beiden Bajonettelemente, wenn der Nocken in der Nische aufgenommen ist, ineinander geschoben und verdreht werden können.

Bei dem Werkzeugkopf kann es sich um einen Ringretraktor handeln, der auch abwinkelbar sein kann. Gerade bei abwinkelbaren Ringretraktoren ist eine effektive Verdrehsicherung der Bajonettverbindung wichtig, da diese eine vergleichsweise lange und schwere Retraktionsstruktur aufweisen, wodurch allein schon durch das Eigengewicht ein vergleichsweise großes Drehmoment auf die Bajonettverbindung ausgeübt wird, das im abgewinkelten Zustand noch höher ausfällt als bei gestreckter Lage. Das mikrochirurgische Instrument weist eine Hülse auf, die drehfest mit der Handhabe verbunden ist, in der die Betätigungsstange längsaxial verschiebbar geführt ist, wobei die Hülse in ihrer Wandung zumindest eine Öffnung aufweisen kann, in die der Klemmstein eingesetzt ist. Damit kann vor dem Schritt d) dann der Schritt c') ausgeführt werden: Einschieben der Betätigungsstange in die Hülse bis die Abplattung an einer Längsachsenposition des Klemmsteins vorliegt, danach Drehen der Betätigungsstange bis der Klemmsteins auf der Abplattung anliegt und zusammen mit der Betätigungsstange Einstecken der Hülse in die Handhabe.

Eine Aufnahmebohrung der Handhabe, in die die Hülse eingeschoben wird, korrespondiert dabei spielarm mit dem Hülsendurchmesser an der Längsachsenposition, an der der Klemmstein vorliegt, wodurch der Klemmstein auch unter Momentenbelastung der Betätigungsstange nicht aus der Öffnung gedrückt wird, sondern eine Verdrehung der Betätigungsstange formschlüssig sperrt.

Der beschriebene Retraktor weist eine proximale Handhabe auf, die mit einem hohlen Schaft verbunden ist, der über eine Bajonettverbindung lösbar mit einer Retraktionsstruktur gekoppelt ist. Die Handhabe weist eine oder mehrere Betätigungsvorrichtung(en) auf, der/die operativ mit einer Betätigungsstange gekoppelt ist/sind, die im hohlen Schaft geführt und operativ mit einem Gelenk der Retraktionsstruktur gekoppelt ist. In einem distalen Endabschnitt des Schafts liegt ein erstes zylindrisches Bajonettelement drehfest vor, das einen in distale Richtung geöffneten längsaxialen Schlitzabschnitt hat. In einem proximalen Anbindungsabschnitt der Retraktionsstruktur liegt ein zweites zylindrisches Bajonettelement drehfest vor, das mit dem ersten Bajonettelement in Eingriff steht und das einen Längsschlitz aufweist, der in einem Sperrzustand der Bajonettverbindung mit dem längsaxialen Schlitzabschnitt des ersten Bajonettelements fluchtet. In dem längsaxialen Schlitzabschnitt des ersten Bajonettelements und in dem Längsschlitz des zweiten Bajonettelements ist ein Nocken längsaxial verschiebbar geführt, der verschiebefest auf der Betätigungsstange angeordnet ist. Die Betätigungsstange hat in ihrem proximalen Endabschnitt eine oder mehrere Abplattung(en). Die Handhabe weist einen oder mehrere Klemmsteine auf, der/die verschiebfest an der Handhabe angeordnet ist und sperrend mit der Abplattung der Betätigungsstange in Eingriff steht/stehen.

Retraktionsstrukturen sind bekannt. Sie weisen mehrere gelenkig verbundene Glieder auf, die zu einem Ring geschlossen werden können, wobei das Glied an dem freien distalen Ende der Retraktionsstruktur einen Kopf hat, mit dem es zum Ring geschlossen werden kann, der unter ein zurückzuhaltendes Organ gelegt werden kann. Abwinklungsmechaniken sind auch bekannt; diese weisen einen feststehenden Teil, mit dem sie mit den Schaft verbunden sind, und einen relativ dazu verschwenkaren zweiten Teil auf, der die Glieder trägt. Zur Betätigung der Schwenkung ist die Betätigungsstange des Retraktor an einen Übertragungsarm bzw. Pleuel angelegt, der wiederum distal mit einem exzentrischen Kraftangriffspunkt des verschwenkbaren zweiten Teils verbunden ist.

Die Betätigungsstange kann genau eine Abplattung, die ein im Querschnitt kreissegmentförmiger Abschnitt sein kann, zwei gegenüberliegende Abplattungen oder polygonal um den Umfang der Betätigungsstange verteilte Abplattungen, die etwa in Form eines regelmäßigen Sechsecks angeordnet sind, aufweisen. Es werden zwei operativ voneinander getrennte Verdrehsicherungen erreicht, die einen sicheren Schutz gegen unbeabsichtigtes Aufdrehen der Bajonettverbindung in jedem Betriebszustand des Retraktors ermöglichen. Die werkzeugnahe Verdrehsicherung, die durch den Eingriff des Nockens in den Längsschlitzen realisiert ist, nimmt ein Drehmoment, das durch das Eigengewicht der Retraktionsstruktur und/oder durch externe Belastung durch ein Organ ausgeübt wird, nahe der Krafteinleitung auf, während die Verdrehsicherung durch den/die Klemmstein(e) eine zusätzliche Verdrehsicherung für den Fall bereit stellt, dass die werkzeugnahe Verdrehsicherung nicht aktiv ist, was der Fall ist, wenn der Nocken noch nicht in die Längsschlitze der beiden Bajonettelemente eingefahren ist. Das erste und/oder das zweite Bajonettelement kann/können als ein integraler Bestandteil des Schafts und/oder des proximalen Endes der Retraktionsstruktur gefertigt werden oder bei der Herstellung des Retraktors zunächst als separates Bauteil gefertigt und an einen Schaftrohling bzw. einen Retraktionsstruktur-Rohling gefügt werden.

In einer weiteren Ausführungsform kann die Betätigungsstange in einem vorbestimmten Arbeitsbereich längsaxial verschiebbar sein und die Abplattung der Betätigungsstange zumindest so lang sein wie der vorbestimmten Arbeitsbereich. Hierdurch wird erst sichergestellt, dass die Betätigungsstange sich über ihren vollen Arbeitsbereich ungehindert bewegen lässt. Zum Koppeln bzw. Entkoppeln der Betätigungsstange mit der durch den oder die Klemmsteine erreichten Verdrehsicherung an der Handhabe können die Klemmsteine jedoch über die Abplattung hinaus abgleiten, wodurch der sperrende Eingriff überwunden werden kann.

In einer noch weiteren Ausführungsform kann die Betätigungsstange einen zumindest entlang eines Umfangsabschnitts umlaufenden Einstich aufweisen. Der Nocken kann einen C-förmigen Fuß haben, der mit den Abmessungen des Einstichs korrespondiert, wobei der Nocken mit dem Fuß in den Einstich der Betätigungsstange eingehängt ist.

Der Nocken kann dabei lediglich formschlüssig in dem Einstich gehalten werden oder zusätzlich mit einem anderen Verbindungsverfahren gehalten werden, etwa mit der Betätigungsstange verschweißt oder verklebt. Die genannten Verbindungsverfahren schränken die Erfindung jedoch nicht ein, vielmehr sind noch andere Befestigungsarten des Nockens möglich.

In noch einer weiteren Ausführungsform kann das zweite Bajonettelement an seinem proximalen Ende zumindest eine Klaue oder auch mehrere Klauen aufweisen. Diese können, müssen aber nicht, in gleichmäßigen Winkelabständen angeordnet sind. Die Klaue(n) erstreckt/erstrecken sich radial nach außen und bilden jeweils einen Eingriffskörper, der mit dem ersten Bajonettelement in Eingriff steht.

Wenn mehrere Klauen zum Einsatz kommen, ist für jede der Klauen ein zugeordneter L-förmiger Schlitz in dem anderen Bajonettelement vorgesehen. Durch zwei oder mehr Klauen kann das übertragbare Drehmoment der Bajonettverbindung gesteigert werden und bei vergleichweise weiten Fertigungstoleranzen die Verkippungsneigung reduziert werden.

Der oder die Klaue(n) können im Sperrzustand der Bajonettverbindung alternativ oder zusätzlich winkelversetzt zum längsaxialen Schlitzabschnitt des ersten Bajonettelements vorliegen. Hierdurch kann sowohl erreicht werden, dass die beiden Längsschlitze der Bajonettelemente im Sperrzustand fluchtend ausgerichtet sind, als auch, dass der Nocken von dem freien distalen Ende des dem Schaft zugeordneten Bajonettelements beim Überschieben des Schafts mitgeschoben werden kann, während der/die Eingriffskörper in den Schlitz/die Schlitze eingeführt werden. Der Bediener des Retraktors muss beim Ankoppeln des Schafts nicht darauf achten, in welcher Position sich der Nocken befindet, der Nocken wird durch das distale Ende des Schafts beim Ankoppeln mit verschoben.

Die Retraktionsstruktur kann gemäß einer weiteren Ausführungsform einen zylindrischen Anbindungsabschnitt haben, mit dem das zweite Bajonettelement verbunden ist. In einem proximalen Endabschnitt des Anbindungsabschnitts liegt dabei eine Nische vor, die mit einer Größe und Position des Nockens korrespondiert, wobei der Nocken in seiner distalen Verschiebeendposition in der Nische aufgenommen werden kann.

Der zylindrische Anbindungsabschnitt der Retraktionsstruktur kann auch als feststehender Teil bezeichnet werden, da er mit dem Schaft verbunden ist und insbesondere auch das Gelenk zur Abwinklung des distalen, beweglichen Teils der Retraktionsstruktur aufweist. Die Nische des proximalen Endabschnitts des Anbindungsabschnitts stellt einen Aufnahmeraum für den Nocken bereit, in dem dieser temporär zur Montage der Bajonettverbindung aufgenommen werden kann, um die Verdrehung der beiden Bajonettelemente nicht zu behindern. In diese Stellung kann er durch eine entsprechende Bewegung der Betätigungsstange nach distal verschoben werden. Es kann an der Handhabe vorgesehen sein, dass der "zusätzliche" Verschiebeweg bis in die Nische erst nach Überwinden einer Sperre erreicht werden kann, um zu verhindern, dass diese Stellung unabsichtlich erreicht wird. Diese Stellung liegt außerhalb des normalen Arbeitsbereichs der Betätigungsstange und wird lediglich zur Montage/Demontage benötigt.

Darüber hinaus kann das zweite Bajonettelement an seinem distalen Ende einen Bund aufweisen, der eine umfängliche Unterbrechung hat. Die Weite der Unterbrechung kann mit der Breite des Nockens korrespondieren, wobei die Unterbrechung eine Fortführung des Längsschlitzes des zweiten Bajonettelements bildet und die Nische etwa durch die Unterbrechung des Bundes gebildet wird. Alternativ oder zusätzlich kann der zylindrische Anbindungsabschnitt der Retraktionsstruktur an seinem proximalen Ende eine radial außen liegende Nase aufweisen, die sich längsaxial nach proximal erstreckt und in die Unterbrechung des Bundes eingreift.

Durch den Eingriff der Nase in der Unterbrechung kann bei der Fertigung eine definierte Winkelstellung der Retraktionsstruktur zugeordneten Bajonett-elements und des Anbindungsabschnitts der Retraktionsstruktur erreicht werden, die es erleichtert, das Bajonettelement korrekt für einen anschließenden Schweißvorgang oder einer Verbindung mittels anderer Fügeverfahren zu positionieren. Daneben kann jedoch auch vorgesehen sein, dass die Nase auch während des Betriebs des Retraktors formschlüssig in die Unterbrechung eingreift, ohne dass das Bajonettelement verschweißt werden muss; hier ist eine Montage des Bajonettelements beispielsweise über Rastmittel oder ähnliche Verbindungselemente möglich. Die Weite und längsaxiale Ausrichtung der Unterbrechung entspricht vorteilhaft der Breite des Längsschlitz des Retraktionsstruktur zugeordneten Bajonettelements; der Längschlitz erstreckt sich quasi weiter nach distal durch den Bund hindurch.

Ferner kann eine Hülse drehfest mit der Handhabe verbunden sein, in der die Betätigungsstange längsaxial verschiebbar geführt ist. Die Hülse kann insbesondere in ihrer Wandung eine oder mehrere Öffnungen aufweisen, in die der/die Klemmstein(e) eingesetzt ist/sind. Die Hülse kann von einer Anpressvorrichtung, etwa von einem Federring, einem Elastomerring und/oder einem elastischen Schlauchstück umgeben sein, die dazu vorgesehen ist, eine nach radial innen weisenden Anpresskraft auf den Klemmstein auszuüben.

Die Außenkontur des Klemmsteins kann vorteilhaft so geformt sein, dass er in eingesetztem Zustand eine "Fortsetzung" des Grundkörperquerschnitts mit sanftem Übergang bildet, sodass die Hülse auch mit eingesetztem Klemmstein in eine korrespondierende Aufnahmebohrung der Handhabe eingesetzt werden kann. Die Hülse kann einen runden oder eckigen Außenquerschnitt haben, was vorteilhaft sein kann, um die Hülse formschlüssig gegen Verdrehen gegenüber der Handhabe zu sichern. Die Hülse ist dazu vorgesehen, bei der Demontage des Retraktors zusammen mit der Betätigungsstange aus der Handhabe entnommen zu werden, da der Eingriff des Klemmsteins in der Abplattung in eingeführtem Zustand nicht überwunden werden kann.

Die radiale Passung des Klemmsteins in der Aufnahmebohrung der Handhabe ist dabei vorteilhaft so eng, das auch bei Rotation der Betätigungsstange der Klemmstein nur unwesentlich radial weggedrückt wird. Bei der Anpressvorrichtung kann es sich neben den beispielhaft genannten auch noch um andere handeln, etwa um ein bandförmiges Zugelement.

Gemäß noch einer weiteren Ausführungsform kann die Hülse in einem proximalen Endabschnitt eine oder mehrere Nut(en) aufweisen, in die zumindest ein korrespondierendes Eingriffsmittel der Handhabe eingreift. Alternativ oder zusätzlich kann die Hülse in einem distalen Endabschnitt eine Kopplungsvorrichtung aufweisen, die mit einem proximalen Ende des Schafts dreh- und verschiebefest lösbar verbunden ist. Die Kopplungsvorrichtung kann beispielsweise eine Mehrzahl von umfänglich angeordneten Längsnuten aufweisen die jeweils in Eingriff mit einer sich nach radial innen erstreckenden Zunge eines Kopplungsstücks stehen, das an einem proximalen Ende des Schafts angeordnet ist.

Die umlaufende Nut in dem proximalen Endabschnitt der Hülse dient der verschiebefesten Kopplung der Hülse mit der Handhabe, wobei entsprechende Eingriffsmittel der Handhabe, beispielsweise radial einfahrende Stifte oder Zungen, in Eingriff mit der umlaufenden Nut gebracht werden. Die Längsnuten am distalen Ende der Hülse sind dazu vorgesehen, zusammen mit korrespondierenden Eingriffsrippen des Kopplungsstücks des Schafts eine verdrehfeste Verbindung von Hülse und Schaft zu ermöglichen. Die Längsnuten können in einer Ausführungsform beispielsweise in gleichmäßigen Winkelabständen angeordnet sein. Das Kopplungsstück des Schafts ist eine Art Kopf bzw. Verbindungsadapter, der eine bequeme Handhabung des Schafts ermöglicht und eine hinreichende Steifigkeit zur Kopplung mit den Nuten hat.

Schließlich können sich die Längsnuten der Kopplungsvorrichtung der Hülse von einem proximalen Ende des Kopplungsstücks des Schaftes weiter nach proximal erstrecken und an ihrem proximalen Ende jeweils zumindest eine Einführschräge haben.

In diese Fortführungen der Längsnuten können wiederum korrespondierende Eingriffskörper, die in der Aufnahmebohrung der Handhabe vorliegen, eingeführt werden, um die Handhabe gegenüber der Hülse und damit indirekt gegenüber der Betätigungsstange gegen Verdrehen zu sichern.

Der distale Endabschnitt der Hülse, in dem sich die Längsnuten erstrecken, kann beispielsweise einen größeren Durchmesser haben als der Rest, wodurch sich zusammen mit den Einführschrägen eine "kronenförmige" Kopplungsvorrichtung ergibt, die, bedingt durch die Einführschrägen, schnell und intuitiv in die Aufnahmebohrung der Handhabe eingeführt werden kann. Diese und weitere Vorteile werden durch die nachfolgende Beschreibung unter Bezug auf die begleitenden Figuren dargelegt. Der Bezug auf die Figuren in der Beschreibung dient der Unterstützung der Beschreibung und dem erleichterten Verständnis des Gegenstands. Die Figuren sind lediglich schematische Darstellungen von Ausführungsbeispielen. Es zeigen:
- **Fig. 1**: eine perspektivische Teilansicht des Retraktors mit der Betätigungsstange in der distalen Verschiebeendposition,
- **Fig. 2**: eine Draufsicht eines Teils des Retraktors mit transparentem Schaft in der Betätigungsstellung der Fig. 1,
- **Fig. 3**: eine perspektivische Teilansicht des Retraktors mit der Betätigungsstange in einer distalen Arbeitsposition,
- **Fig. 4**: eine Draufsicht eines Teils des Retraktors mit transparentem Schaft in der Betätigungsstellung der Fig. 3,
- **Fig. 5a**: eine Längsschnittansicht eines Teils des Retraktors ohne Bajonetthülse und Schaft in der Betätigungsstellung der Fig. 3,
- **Fig. 5b**: eine Querschnittansicht des Schafts des Retraktors,
- **Fig. 6**: eine perspektivische Teilansicht des Retraktors mit der Betätigungsstange in einer mittleren Arbeitsposition,
- **Fig. 7**: eine Draufsicht eines Teils des Retraktors mit transparentem Schaft in der Betätigungsstellung der Fig. 6,
- **Fig. 8**: eine perspektivische Ansicht des proximalen Endabschnitts der Betätigungsstange,
- **Fig. 9a,b**: perspektivische Ansichten des distalen Endes des Retraktors mit geschlossener Retraktionsstruktur,
- **Fig. 10**: eine perspektivische Teilansicht des proximalen Endes des Retraktors mit ausgeblendeter Handhabe,
- **Fig. 11**: einen Längsschnitt der in Fig. 10 gezeigten Ansicht,
- **Fig. 12a,b**: Querschnittsansichten des proximalen Endes des Retraktors mit ausgeblendeter Handhabe,
- **Fig. 13a**: eine perspektivische Ansicht des Retraktors mit der Betätigungsstange in der distalen Verschiebeendposition,
- **Fig. 13b**: eine perspektivische Ansicht des Retraktors mit der Betätigungsstange in einer mittleren Arbeitsposition.

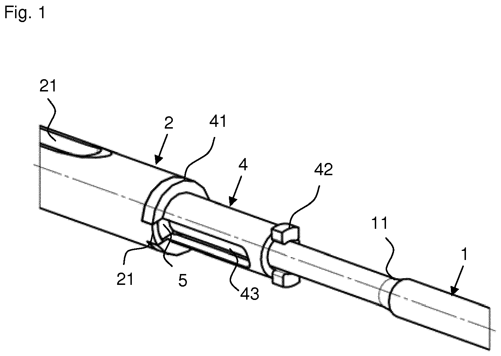

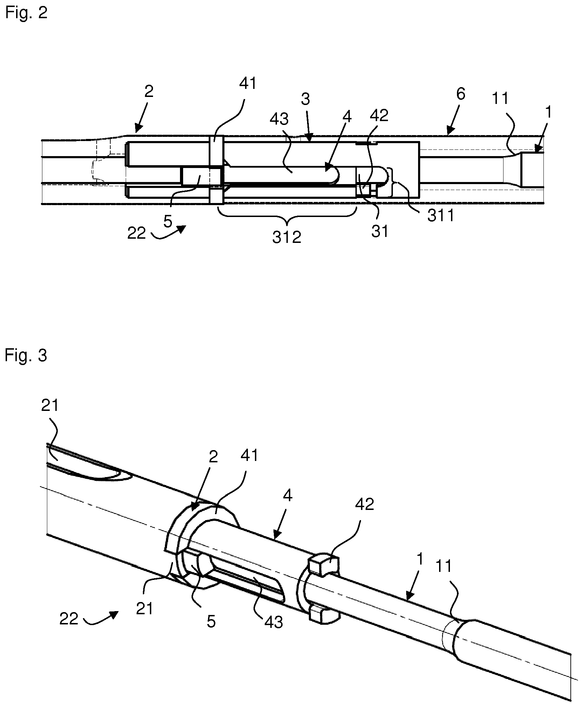

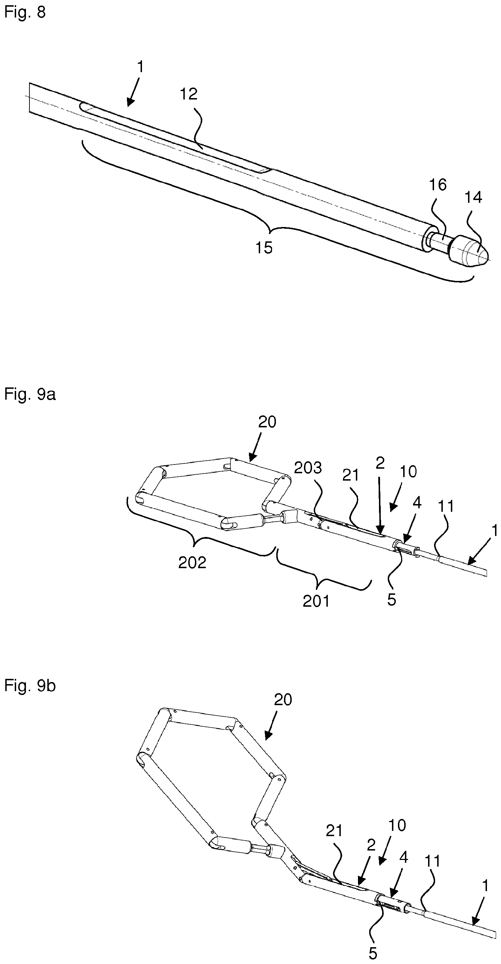

Die perspektivische Teilansicht des Retraktors aus **Fig.1** zeigt einen Abschnitt um die Bajonettverbindung, über die die Retraktionsstruktur 20 (siehe **Fig. 9a****, b**) an den Schaft 6 (siehe **Fig. 2**) gekoppelt ist. Es ist die Betätigungsstange 1 zu erkennen, die das eigentliche Übertragungsglied von der Handhabe zur Retraktionsstruktur 20 darstellt. Die Betätigungsstange 1 hat eine Verjüngung 11, wobei der Durchmesser der Betätigungsstange 1 in ihrem proximalen Teil (in der Figur rechts) größer als in ihrem distalen Abschnitt (in der Figur links) ist. Hierdurch ist es möglich, einen vergleichsweise bauraumsparenden Werkzeugkopf zu realisieren, während die Torsionssteifigkeit in den proximalen Abschnitten sichergestellt ist.

In der Abbildung ist die Retraktionsstruktur 20 nicht dargestellt, diese befindet sich in einem Bereich, der links außerhalb des dargestellten Bereichs liegt, jedoch ist der schaftförmige Anbindungsabschnitt 2 der Retraktionsstrukur 20 gezeigt, von dem sich das Bajonettelement 4 nach proximal (in der Abbildung rechts) erstreckt. Das Bajonettelement 4 ist mit dem Anbindungsabschnitt 2 verschweißt, wobei zur erleichterten Fertigung der Bajonetteinsatz 4 einen Bund 41 hat, der an dem proximalen Ende des Anbindungsabschnitts 2 anliegt. Der Bund 41 hat eine Unterbrechnung bzw. Aussparung, in die eine Nase 21 des Anbindungsabschnitts 2 eingreift, um eine vorbestimmte Winkelposition bei der Montage vorzugeben. Am proximalen Ende hat das Bajonettelement 4 zwei gegenüberliegende Klauen 42, die sich nach radial außen erstrecken und die mit einem anderen Bajonettelement 3 (siehe Fig. 2) in Eingriff gebracht werden können. Das "innere", dem Anbindungsabschnitt 2 zugeordnete Bajonettelement 4 wird als zweites Bajonettelement 4 und das "äußere", dem Schaft 6 zugeordnete Bajonettelement als erstes Bajonettelement 3 (siehe **Fig. 2**) bezeichnet.

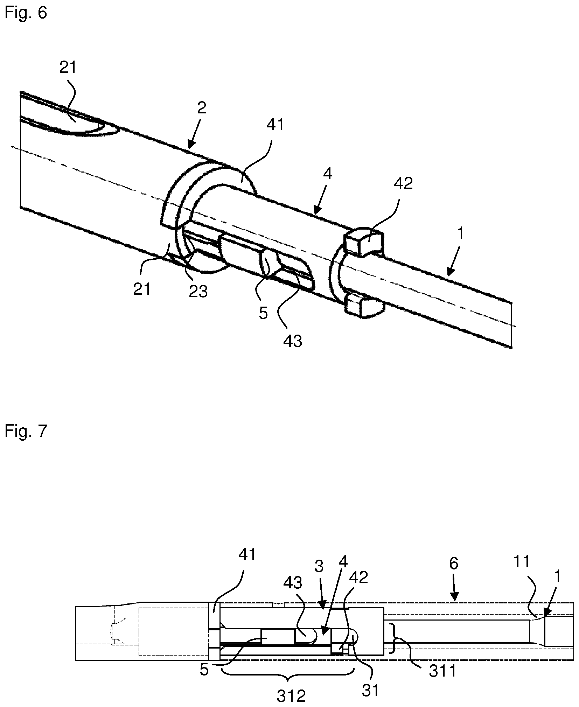

Auf der Betätigungsstange 1 ist verschiebefest ein Nocken 5 geführt, der in der gezeigten Betätigungsstellung der Betätigungsstange 1 in einer Nische 23 (siehe **Fig. 6**) aufgenommen ist, die sich zwischen der Nase 21 und der Unterbrechung des Bunds 41 des zweiten Bajonettelements 4 befindet. Bei einer Bewegung der Betätigungsstange 1 in proximale Richtung wird der Nocken 5 zwangsgekoppelt aus der Nische 23 heraus bewegt und entlang des Längsschlitzes 43, der in der Wandung des zweiten Bajonettelements 4 vorliegt, verfahren (siehe dazu **Fig. 3** bis **Fig. 7**).

In **Fig. 2** ist der in **Fig. 1** gezeigte Teil des Retraktors in einer Draufsicht dargestellt, wobei sich die Betätigungsstange 1 und damit auch der Nocken 5 in der gleichen distalen Verschiebeendposition befinden wie in **Fig. 1****.** Hier ist jedoch der Schaft 6 und der Anbindungsabschnitt 2 der Retraktionsstruktur transparent dargestellt, so dass die Führung der Bajonettelemente 3,4 ineinander zu erkennen ist. Von dem Bund 41 des zweiten Bajonettelements 4 erstreckt sich ein Einführabschnitt nach distal (in der Figur links), mit dem das zweite Bajonettelement 4 in dem Anbindungsabschnitt geführt ist. Der Längsschlitz 43 erstreckt sich nach distal sowohl durch den Bund als auch durch den Einführabschnitt. Das erste Bajonettelement 3 weist einen L-förmigen Schlitz 31 auf, der einen längsaxialen Abschnitt 312 und einen umfänglichen Abschnitt 311 hat. Der Schlitz 31 hat eine Weite, die so dimensioniert ist, dass sowohl die Klauen 42 des zweiten Bajonettelements 4 als auch der Nocken 5 darin aufgenommen werden können.

Das erste Bajonettelement 3 ist drehfest mit dem Schaft 6 verbunden und bildet zusammen mit dem zweiten Bajonettelement die Bajonettverbindung zur Ankopplung des Schafts 6 an den Anbindungsabschnitt der Retraktionsstruktur.

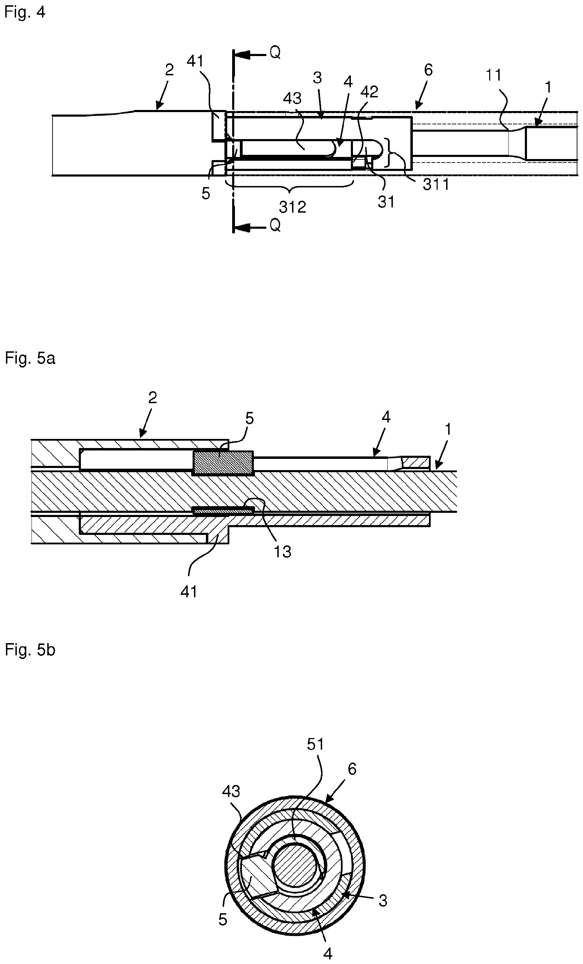

In den **Fig. 3** und **Fig. 4** ist jeweils eine perspektivische Ansicht und eine Draufsicht des Abschnitts des Retraktors um die Bajonettverbindung dargestellt, wobei die Betätigungsstange 1 ein Stück weit nach proximal verfahren ist; sie befindet sich nicht mehr in der distalen Verschiebeendposition sondern in einer Arbeitsposition. Der Nocken 5 ist in dieser Stellung nicht mehr vollständig in der Nische 23 (siehe **Fig. 6**) aufgenommen, sondern tritt ein Stück heraus. Die radiale Ausdehnung des Nockens 5 ist so dimensioniert, dass er sich nicht nur durch das zweite (innere) Bajonettelement 2 erstreckt, sondern auch in den längsaxialen Abschnitt 312 des Schlitzes 31 des ersten Bajonettelements 3 eingreift, wodurch eine Verdrehsicherung der beiden Bajonettelemente 3,4 gegeneinander erreicht wird.

**Fig. 5a** zeigt einen Schnitt des Retraktors bezüglich der Mittellängsebene, wobei der Schaft 6 ausgeblendet ist. Die Verbindung des Nockens 5 mit der Betätigungsstange 1 ist hierin gut zu erkennen. Die Betätigungsstange 1 weist einen umlaufenden Einstich 13 auf, auf die der Nocken 5 mit einem Fuß aufgesteckt ist. Der Fuß 51 ist C-förmig, was in der Querschnittsansicht der
**Fig. 5b** dargestellt ist, wobei die Schnittebene Q in der **Fig. 4** eingezeichnet ist. Die Dicke des C-förmigen Fußes 51 entspricht dabei maximal der Tiefe des Einstichs 13.

**Fig. 6** und **Fig. 7** zeigen jeweils eine perspektivische Teilansicht des schon in den **Fig. 1** bis **Fig. 4** gezeigten Ausschnitts um die Bajonettverbindung.

Die Betätigungsstange 1 befindet sich in einer mittleren Arbeitsposition, wobei der Nocken 5 hier vollständig aus der Nische 23 heraus getreten ist und seine sperrende Funktion ausüben kann, da er gleichzeitig in das erste (äußere) Bajonettelement 3 und das zweite (innere) Bajonettelement 4 eingreift.

Bei den in **Fig.1** und **Fig. 2** gezeigten Verschiebestellungen der Betätigungsstange 1 bzw. des Nockens 5 könnte die Bajonettverbindung unbeabsichtigt aufgedreht werden, da der Nocken 5 noch nicht sperrend in die beiden Längsschlitze 312, 43 des ersten 4 und zweiten Bajonettelements 3 eingreift. Das proximale Ende des Retraktors 10 mit angekoppelter Retraktionsstruktur 20 ist in den **Fig. 9a** und **Fig. 9b** dargestellt, wobei in **Fig. 9a** sich die Betätigungsstange 1 in der Verschiebestellung der **Fig. 1** befindet und in **Fig. 9b** in der Verschiebestellung der **Fig. 3****.** Die Betätigungsstange 1 ist hierbei an einem Pleuel bzw. Übertragungsarm angelenkt, das/der exzentrisch mit einem Krafteinleitungspunkt des verschwenkbaren distalen Teils 202 der Retraktionsstruktur 20 verbunden ist, während der Anbindungsabschnitt 2 den feststehende Teil 201 der Retraktionsstruktur 20 bildet. Der verschwenkbare Teil 202 und der feststehenden Teil 201 sind über das Gelenk 203 verbunden, dessen Gelenkachse die Schwenkachse der beiden Teile 201,202 der Retraktionsstruktur 20 bildet. Das Pleuel bzw. der Übertragungsarm ist in der gestreckten Lage der Retraktionsstruktur 20 in einem Schlitz 21 aufgenommen, so dass der Retraktor 10 in dieser Stellung sicher in den Körper eingeführt werden kann.

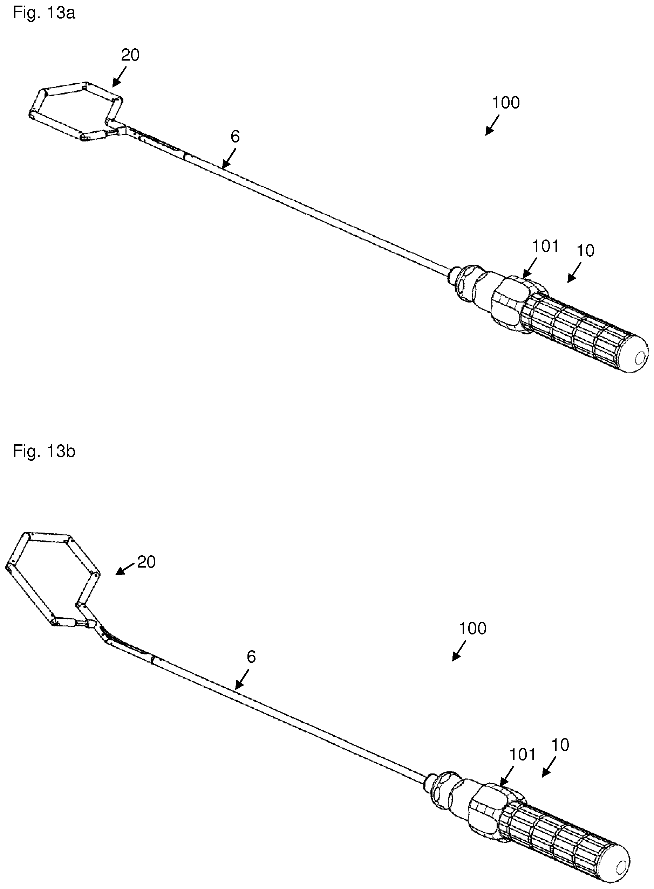

Um ein unbeabsichtiges Aufdrehen der Bajonettverbindung auch in der gestreckten Stellung zu verhindern, wird eine handhabenahe Verdrehsicherung vorgeschlagen, die formschlüssig auf die Abplattung 12 der Betätigungsstange 1 einwirkt, wobei die Abplattung 12 im proximalen Endabschnitt 15 der Betätigungsstange vorliegt, was in **Fig. 8** gezeigt ist. Der proximale Endabschnitt 15 der Betätigungsstange 1 ist dazu vorgesehen, in die Handhabe 10 (siehe **Fig. 13a****,b**) eingesteckt zu werden. Die auf die Betätigungsvorrichtung 101 (siehe **Fig, 13a****,b**) ausgeübten Betätigungskräfte werden bei Druckkräften auf den pilzförmigen Kopf 14 und bei Zugkräften auf die Einschnürung 16 ausgeübt, während die Betätigungsstange 1 über die Abplattung 12, die in Eingriff einem Klemmstein gebracht wird, der verschiebefest an der Handhabe 10 angeordnet ist, verdrehsicher bezüglich der Handhabe 10 gehalten wird.

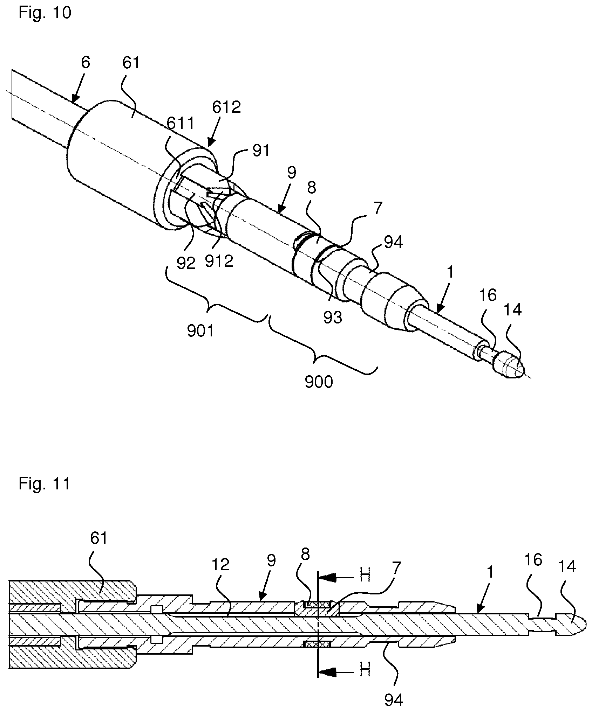

Diese handhabenahe Verdrehsicherung ist in den **Fig. 10** bis **Fig. 12b** gezeigt. Die Hülse 9 ist dabei dazu vorgesehen, dreh- und verschiebefest mit der Handhabe 10 (siehe **Fig. 13a,b****)** verbunden zu werden.

Zur Sperrung der Axialbewegung der Hülse 9 gegenüber der handhabe hat die Hülse 9 eine umlaufende Nut 94, die in Eingriff mit einem oder mehreren korrespondierenden Eingrifsmittel(n) der Handhabe gebracht werden kann, während zur Sicherung des Verdrehfreiheitsgrades die längsaxial verlaufenden Nuten 92 in dem distalen Endabschnitt 901 der Hülse 9 vorgesehen sind. Die Nuten 92 sind hierbei in einem Kopfbereich angeordnet, dessen Durchmesser größer ist als der Durchmesser der Hülse 9 in den restlichen Bereichen. Die Längsnuten 92 sind in gleichmäßigen Winkelabständen um den Umfang verteilt, wobei die Stege 91 zwischen den einzelnen Nuten 92 angeschrägt sind, um jeweils eine Einführschräge 912 zu bilden. Die Einführschrägen 912 ermöglichen es, die Hülse 9 einfacher mit der Handhabe in Eingriff zu bringen, da die Einführschrägen 912 beim in Kontakt bringen mit den korrespondierenden Eingriffsrippen der Handhabe diese selbsttätig in die zur Kopplung geeignete Winkelposition drehen. Der Schaft 6 ist lösbar mit der Hülse 9 verbunden, wobei zur Winkelfestlegung die gleichen Nuten 92 Verwendung finden, wie sie auch für die drehfeste Kopplung mit der Handhabe verwendet werden. Der Schaft 6 hat an seinem proximalen Ende ein Kopplungsstück 61, das an seinem proximalen Ende nach innen ragende Zungen 611 aufweist, die jeweils sperrend in eine der Nuten 92 am "Kopf" der Hülse eingreifen, wobei sich die Nuten unter dem Kopplungsstück 61 noch weiter nach distal erstrecken.

Die Betätigungsstange 1 ist in der Hülse 9 geführt, wobei die Abplattung 12 in dem Bereich unter der Öffnung 93 liegt. In die Öffnung 93 ist ein Klemmstein 7 eingesetzt, dessen Innenseite plan ist und auf der Abplattung 12 der Betätigungsstange 1 anliegt. Um die Hülse 9 und den Klemmstein 7 herum ist umlaufend eine Anpressvorrichtung 8 geführt, bei der es sich um ein Gummiband handelt. Die Anpressvorrichtung 8 übt eine nach radial innen gerichtete Kraft auf den Klemmstein 7 aus, wodurch dieser auf die Abplattung 12 gedrückt wird bzw. auch in nicht an die Handhabe gekoppeltem Zustand verliersicher in der Öffnung 93 aufgenommen ist. Bei in die Handhabe eingeführtem Zustand hat die Hülse 9 bzw. der Klemmstein 7 nur sehr wenig Radialspiel in der korrespondierenden Aufnahmebohrung der Handhabe, wodurch der Klemmstein 7 auch bei Drehmomentbelastung der Betätigungsstange 1 nicht nach radial außen weggedrückt werden kann, sondern sperrend mit seinen Flanken auf den Seitenflächen der Öffnung 93 der Hülse 9 anliegt.

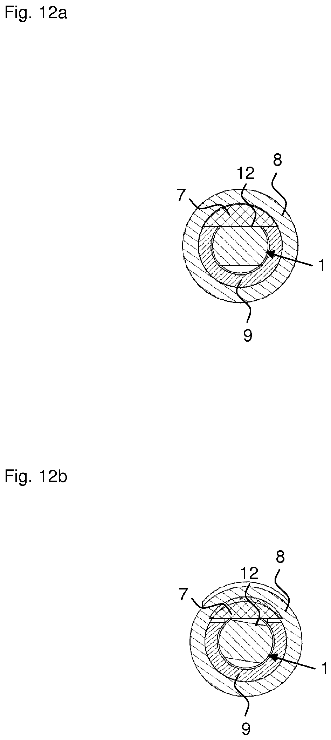

In den **Fig.12a** und **Fig. 12b** ist der Schnitt gemäß der Schnittebene H (siehe **Fig. 11**) gezeigt, wobei der Klemmstein 7 in **Fig. 12a** in seinem Sperrzustand vorliegt und in **Fig. 12b** im Lösezustand. Damit die Betätigungsstange 1 von der Hülse 9 getrennt werden kann, muss diese schon von der Handhabe entkoppelt sein, da der Klemmstein 7 ansonsten keine radiale Bewegung ausführen kann. Um die Betätigungsstange 1 aus der Hülse 9 zu entnehmen, muss diese um ihre Längsachse gedreht werden, wodurch der Klemmstein 7 nach radial außen bewegt und der Eingriff mit der Abplattung 12 aufgehoben wird. Infolgedessen kann die Betätigungsstange 1 aus der Hülse 9 herausgezogen werden. Zum Zusammenbau muss die Betätigungsstange 1 zunächst in die Hülse 9 eingeführt werden und eventuell verdreht werden, bis der Klemmstein 7 mit seiner Innenseite auf der Abplattung 12 der Betätigungsstange 1 aufliegt, sodass der Klemmstein so weit nach radial innen in die Öffnung aufgenommen wird, dass seine Außenkontur bündig mit der Außenkontur der Hülse 9 ist.

Der zusammengebaute Retraktor 100 ist in den **Fig. 13a** und **Fig. 13b** dargestellt. Im gestreckten Zustand der **Fig. 13a** ist nur die handhabenahe Verdrehsicherung im Eingriff und verhindert ein unbeabsichtigtes Aufdrehen der Bajonettverbindung, während im abgewinkelten Zustand der **Fig. 13b** auch der Eingriff des Nockens 5 in die beiden Längsschlitze 312,43 der Bajonettelemente 3,4 zur Verdrehsicherung der Bajonettverbindung beiträgt. Das Betätigungselement 101 der Handhabe 10 ist hier als Drehrad 101 ausgebildet und ist zur Erzeugung einer Abwinkelbewegung der Retraktionsstruktur 20 operativ mit der Betätigungsstange gekoppelt.

## Patentansprüche

1. Montageverfahren für ein mikrochirurgisches Instrument, das eine proximale Handhabe (10) aufweist, die mit einem hohlen Schaft (6) mechanisch verbindbar ist, der über eine Bajonettverbindung lösbar mit einem Werkzeugkopf (20) koppelbar ist, wobei die Handhabe (10) zumindest eine Betätigungsvorrichtung (101) aufweist, die operativ mit einer Betätigungsstange (1) koppelbar ist, die in dem Schaft (6) geführt werden kann, wobei die Bajonettverbindung zwei Bajonettelemente (3,4) mit je einem Längsschlitz (312,43) aufweist, die in einem Sperrzustand der Bajonettverbindung fluchten, und in denen zumindest ein längsaxial verschiebbarer Nocken (5) geführt ist, und wobei die Betätigungsstange (1) mit ihrem proximalen Endabschnitt (15) in die Handhabe einsetzbar ist und zumindest eine Abplattung (12) aufweist, mit der zumindest ein Klemmstein (7), der bezüglich der Längsachse verschiebfest an der Handhabe (10) angeordnet ist, in Eingriff gebracht werden kann, wobei eine mit dem Schaft lösbar verbundene Hülse vorgesehen ist,
dreh- und verschiebefest mit der Handhabe verbunden zu werden und die Betätigungsstange in der Hülse geführt werden kann, und wobei zur Sperrung der Axialbewegung der Hülse gegenüber der Handhabe die Hülse eine umlaufende Nut hat, die in Eingriff mit einem oder mehreren korrespondierenden Eingriffsmittel(n) der Handhabe gebracht werden kann, während zur Sicherung des Verdrehfreiheitsgrades längsaxial verlaufende Nuten in dem distalen Endabschnitt der Hülse vorgesehen sind, umfassend die Schritte:
a) Bereitstellen der Handhabe (10), des hohlen Schafts (6) und des Werkzeugkopfs (20) mit operativ daran gekoppelter Betätigungsstange (1),
b) Schieben des Schafts (6) mit einem Bajonettelement (3,4) voraus über die Betätigungsstange (1) und in Eingriff Bringen des Bajonettelements (3,4) des Schafts (6) mit dem anderen Bajonettelement (3,4),
c) relativ zueinander Verdrehen des Schafts (6) und des Werkzeugkopfs (20), bis die Bajonettverbindung in dem Sperrzustand vorliegt, dadurch auch fluchtend Ausrichten der Längsschlitze (312,43) der Bajonettelemente (3,4),
d) Einstecken des proximalen Endabschnitts (15) der Betätigungsstange (1) in die Handhabe (10), dabei in Eingriff Bringen der Abplattung (12) der Betätigungsstange (1) mit dem Klemmstein (7) der Handhabe (10) und Sichern der Betätigungsstange (1) gegen Verdrehen gegenüber der Handhabe,
e) mechanisches Verbinden der Handhabe (10) mit der Hülse,
f) Verschieben des Nockens (5) in die Längsschlitze (312,43) und Ausbilden einer werkzeugnahen Verdrehsicherung.

2. Montageverfahren nach Anspruch 1, wobei der Nocken (5) verschiebefest mit der Betätigungsstange (1) verbunden ist und in dem Schritt f) die Betätigungsvorrichtung (101) der Handhabe (10) betätigt und dadurch die Betätigungsstange (1) längsaxial bewegt und der Nocken (5) zwangsgekoppelt mitbewegt wird.

3. Montageverfahren nach Anspruch 1 oder 2, wobei die Längsschlitze (312,43) der Bajonettelemente (3,4) und zumindest ein Eingriffskörper (42) der Bajonettverbindung in einem Freigabezustand der Bajonettverbindung angular versetzt vorliegen, und wobei in dem Schritt b) ein distales Ende eines dem Schaft (6) zugeordneten Bajonettelements (3,4) an dem Nocken (5) zur Anlage gebracht wird, und der Nocken (5) beim Verschieben des Schafts (6) mitverschoben wird.

4. Montageverfahren nach zumindest einem der Ansprüche 1 bis 3, wobei an einem proximalen Ende (22) des Werkzeugkopfs (20) ein Anbindungsabschnitt (2) vorliegt, der eine Nische (23) aufweist, die mit einer Größe und Position des Nockens (5) korrespondiert, und wobei vor dem Schritt c) der Schritt b') Verschieben des Nockens (5) in eine distale Verschiebeendposition, dort Aufnahmen des Nockens (5) in der Nische (23) und dadurch Freigeben eines Verdrehfreiheitsgrads der Bajonettelemente (3,4), ausgeführt wird.

5. Montageverfahren nach zumindest einem der Ansprüche 1 bis 4, wobei der Werkzeugkopf (2) ein Ringretraktor ist, bevorzugt ein abwinkelbarer Ringretraktor.

6. Montageverfahren nach zumindest einem der Ansprüche 1 bis 5 wobei die Hülse (9) in ihrer Wandung zumindest eine Öffnung (93) aufweist, in die der Klemmstein (7) eingesetzt ist, und wobei vor dem Schritt d) der Schritt c') Einschieben der Betätigungsstange (1) in die Hülse (9) bis die Abplattung (12) an einer Längsachsenposition des Klemmsteins (7) vorliegt, Drehen der Betätigungsstange (1) bis der Klemmsteins (7) auf der Abplattung (12) anliegt und zusammen mit der Betätigungsstange (1) Einstecken der Hülse (9) in die Handhabe (10), ausgeführt wird.

## Claims

1. Assembly method for a microsurgical instrument, which has a proximal handle (10) mechanically connectable to a hollow shaft (6), which shaft (6) can be coupled releasably to a tool head (20) via a bayonet connection, wherein the handle (10) has at least one actuation device (101), which can be operatively coupled to an actuation rod (1) that can be guided in the shaft (6), wherein the bayonet connection has two bayonet elements (3, 4) with respective longitudinal slits (312, 43) which are aligned in a locked state of the bayonet connection, and at least one longitudinally movable cam (5) is guided axially in the longitudinal slits (312, 43), and wherein the actuation rod (1) can be inserted with its proximal end portion (15) into the handle and has at least one flattened part (12), which can be engaged by at least one clamping block (7) arranged on the handle (10) in a fixed position with respect to the longitudinal axis, wherein a sleeve connected releasably to the shaft is provided in order to be connected to the handle for conjoint rotation and movement therewith, and the actuation rod can be guided in the sleeve, and wherein, in order to lock the axial movement of the sleeve with respect to the handle, the sleeve has a circumferential groove that can be brought into engagement with one or more corresponding engagement means of the handle, while, in order to secure the degree of freedom in rotation, grooves extending along the longitudinal axis are provided in the distal end portion of the sleeve, said method comprising the steps of:
a) making available the handle (10), the hollow shaft (6) and the tool head (20), with the actuation rod (1) operatively coupled thereon,
b) pushing the shaft (6), with a bayonet element (3, 4) at the front, over the actuation rod (1) and bringing the bayonet element (3, 4) of the shaft (6) into engagement with the other bayonet element (3, 4),
c) rotating the shaft (6) and the tool head (20) relative to each other until the bayonet connection is in the locked state, thereby also aligning the longitudinal slits (312, 43) of the bayonet elements (3, 4),
d) inserting the proximal end portion (15) of the actuation rod (1) into the handle (10), thereby engaging the flattened part (12) of the actuation rod (1) with the clamping block (7) of the handle (10) and securing the actuation rod (1) against rotation relative to the handle,
e) mechanically connecting the handle (10) to the sleeve,
f) moving the cam (5) into the longitudinal slits (312, 43) and forming an anti-rotation means near the tool.

2. Assembly method according to Claim 1, wherein the cam (5) is connected to the actuation rod (1) for conjoint movement therewith and, in step f), the actuation device (101) of the handle (10) is actuated and, in this way, the actuation rod (1) is moved along the longitudinal axis and the cam (5) is necessarily carried along with it.

3. Assembly method according to Claim 1 or 2, wherein the longitudinal slits (312, 43) of the bayonet elements (3, 4) and at least one engagement body (42) of the bayonet connection are at an angular offset in a release state of the bayonet connection, and wherein, in step b), a distal end of a bayonet element (3, 4) assigned to the shaft (6) is brought to bear on the cam (5), and the cam (5) is carried along in the movement of the shaft (6).

4. Assembly method according to at least one of Claims 1 to 3, wherein, at a proximal end (22) of the tool head (20), an attachment portion (2) is present which has a niche (23) that corresponds to a size and position of the cam (5), and wherein, before step c), step b') is carried out, i.e. moving the cam (5) to a distal end position of movement, there receiving the cam (5) in the niche (23) and thereby releasing a degree of freedom of rotation of the bayonet elements (3, 4).

5. Assembly method according to at least one of Claims 1 to 4, wherein the tool head (2) is a ring retractor, preferably a pivotable ring retractor.

6. Assembly method according to at least one of Claims 1 to 5, wherein the sleeve (9) has, in its wall, at least one opening (93) in which the clamping block (7) is fitted, and wherein, before step d), step c') is carried out, i.e. pushing the actuation rod (1) into the sleeve (9) until the flattened part (12) is present at a longitudinal axis position of the clamping block (7), turning the actuation rod (1) until the clamping block (7) bears on the flattened part (12), and, together with the actuation rod (1), inserting the sleeve (9) into the handle (10).

## Revendications

1. Procédé de montage pour un instrument microchirurgical, qui présente une poignée proximale (10) qui peut être reliée mécaniquement à un arbre creux (6) qui peut être accouplé de manière détachable à une tête d'outil (20) par le biais d'un raccord à baïonnette, la poignée (10) présentant au moins un dispositif d'actionnement (101) qui peut être accouplé fonctionnellement à une tige d'actionnement (1) qui peut être guidée dans l'arbre (6), le raccord à baïonnette présentant deux éléments de baïonnette (3, 4) ayant chacun une fente longitudinale (312, 43), lesquelles sont en alignement dans un état de verrouillage du raccord à baïonnette, et dans lesquelles est guidée au moins une came (5) pouvant être déplacée axialement longitudinalement et la tige d'actionnement (1) pouvant être insérée dans la poignée par sa portion d'extrémité proximale (15), et présentant au moins un méplat (12) avec lequel peut s'engager au moins une pièce de serrage (7), qui est disposée dé manière non déplaçable par rapport à l'axe longitudinal sur la poignée (10), une douille connectée de manière amovible à l'arbre étant prévue de manière à être connectée à la poignée de manière solidaire en rotation et non coulissante et la tige d'actionnement pouvant être guidée dans la douille, et, pour le verrouillage du coulissement axial de la douille par rapport à la poignée, la douille présentant une rainure périphérique qui peut être amenée en prise avec un ou plusieurs moyens d'engagement correspondants de la poignée, tandis que pour la fixation du degré de liberté de rotation, des rainures s'étendant axialement longitudinalement sont prévues dans la portion d'extrémité distale de la douille, le procédé comprenant les étapes suivantes :
a) fourniture de la poignée (10), de l'arbre creux (6) et de la tête d'outil (20) avec la tige d'actionnement (1) accouplée fonctionnellement à celle-ci,
b) coulissement de l'arbre (6) avec un élément de baïonnette (3, 4) vers l'avant par-dessus la tige d'actionnement (1) et engagement de l'élément de baïonnette (3, 4) de l'arbre (6) avec l'autre élément de baïonnette (3, 4),
c) rotation mutuelle de l'arbre (6) et de la tête d'outil (20) jusqu'à ce que le raccord à baïonnette soit dans l'état verrouillé, et de ce fait également, orientation avec alignement des fentes longitudinales (312, 43) des éléments de baïonnette (3, 4),
d) insertion de la portion d'extrémité proximale (15) de la tige d'actionnement (1) dans la poignée (10), pour ainsi amener en prise le méplat (12) de la tige d'actionnement (1) avec la pièce de serrage (7) de la poignée (10) et fixer la tige d'actionnement (1) pour l'empêcher de tourner par rapport à la poignée,
e) connexion mécanique de la poignée (10) à la douille,
f) coulissement de la came (5) dans les fentes longitudinales (312, 43) et réalisation d'une fixation en rotation proche de l'outil.

2. Procédé de montage selon la revendication 1, dans lequel la came (5) est connectée de manière non coulissante avec la tige d'actionnement (1) et, dans l'étape f), le dispositif d'actionnement (101) actionne la poignée (10) et de ce fait la tige d'actionnement (1) est déplacée axialement longitudinalement et la came (5) est déplacée conjointement par accouplement forcé.

3. Procédé de montage selon la revendication 1 ou 2, dans lequel les fentes longitudinales (312, 43) des éléments de baïonnette (3, 4) et au moins un corps d'engagement (42) du raccord à baïonnette sont disposés de manière décalée angulairement dans un état de libération du raccord à baïonnette, et dans lequel, dans l'étape b), une extrémité distale d'un élément de baïonnette (3, 4) associée à l'arbre (6) est amenée en appui contre la came (5), et la came (5) est déplacée conjointement lors du déplacement de l'arbre (6).

4. Procédé de montage selon au moins l'une quelconque des revendications 1 à 3, dans lequel, au niveau d'une extrémité proximale (22) de la tête d'outil (20), est disposée une portion de liaison (2) qui présente une niche (23) qui correspond à une dimension et une position de la came (5) et dans lequel, avant l'étape c), est réalisée l'étape b') de déplacement de la came (5) dans une position de fin de course de déplacement distal, avec réception à cet endroit de la came (5) dans la niche (23) et de ce fait libération d'un degré de liberté de rotation des éléments de baïonnette (3, 4) .

5. Procédé de montage selon au moins l'une quelconque des revendications 1 à 4, dans lequel la tête d'outil (2) est un rétracteur annulaire, de préférence un rétracteur annulaire pouvant être coudé.

6. Procédé de montage selon au moins l'une quelconque des revendications 1 à 5, dans lequel la douille (9) présente, dans sa paroi, au moins une ouverture (93) dans laquelle est insérée la pièce de serrage (7) et dans lequel avant l'étape d), est réalisée l'étape c') d'insertion de la tige d'actionnement (1) dans la douille (9) jusqu'à ce que le méplat (12) se trouve au niveau d'une position d'axe longitudinal de la pièce de serrage (7), de rotation de la tige d'actionnement (1) jusqu'à ce que la pièce de serrage (7) s'applique contre les méplats (12) et conjointement avec la tige d'actionnement (1), d'insertion de la douille (9) dans la poignée (10).
